# EUROPEAN PATENT SPECIFICATION

(11) **EP 3 600 396 B1**
(45) Date of publication and mention of the grant of the patent: **09.12.2026**
(21) Application number: 18777243.9
(22) Date of filing: 30.03.2018
(51) Int. Cl.: A61K 9/00, A61K 39/00, A61K 45/06, A61K 47/20, C07K 14/705

(54) **NUCLEIC ACID VACCINE COMPOSITION COMPRISING A LIPID FORMULATION, AND METHOD OF INCREASING THE POTENCY OF NUCLEIC ACID VACCINES**
NUKLEINSÄUREIMPFSTOFFZUSAMMENSETZUNG MIT EINER LIPIDFORMULIERUNG UND VERFAHREN ZUR ERHÖHUNG DER POTENZ VON NUKLEINSÄUREIMPSTOFFEN
COMPOSITION VACCINALE D'ACIDE NUCLÉIQUE COMPRENANT UNE FORMULATION LIPIDIQUE, ET PROCÉDÉ PERMETTANT D'AUGMENTER L'EFFICACITÉ DE VACCINS D'ACIDE NUCLÉIQUE

(30) Priority: 30.03.2017 US 201762478875 P
(43) Date of publication of application: 05.02.2020
(73) Proprietor: The Government of the United States of America as represented by the Secretary of the Army, Fort Detrick, MD 21702 (US); Arcturus Therapeutics, Inc., San Diego, CA 92121 (US)
(72) Inventor: HOOPER, Jay, W., New Market, MD 21774 (US); MUCKER, Eric, M., Thurmont, MD 21788 (US); CHIVUKULA, Padmanabh, San Diego, CA 92121 (US)
(74) Representative: J A Kemp LLP
(86) International application number: PCT/US2018/025482
(87) International publication number: WO 2018/183901

(56) References cited:
- WO-A1-2016/081029
- WO-A1-2017/019523
- US-A1- 2006 223 769
- US-A1- 2009 263 407
- US-A1- 2014 242 152
- US-A1- 2015 141 678
- US-A1- 2015 239 834
- SUVASINI RAMASWAMY ET AL: "Systemic delivery of factor IX messenger RNA for protein replacement therapy", PROCEEDINGS OF THE NATIONAL ACADEMY OF SCIENCES, vol. 114, no. 10, 7 March 2017 (2017-03-07), pages E1941 - E1950, XP055756678, ISSN: 0027-8424, DOI: 10.1073/pnas.1619653114
- H OYA ALPAR ET AL: "Strategies for DNA vaccine delivery", EXPERT OPINION ON DRUG DELIVERY, vol. 2, no. 5, 16 September 2005 (2005-09-16), GB, pages 829 - 842, XP055756928, ISSN: 1742-5247, DOI: 10.1517/17425247.2.5.829

## Description

### FIELD OF THE INVENTION

This present invention relates to a method to enhance the potency of plasmid-based nucleic acid vaccines and immunotherapies, composition therefor, and a composition containing a vaccine and lipid formulation with enhanced potency. In particular, purified plasmid nucleic acid encoding vaccine immunogens, or immune response molecules, are combined in a lipid formulation. This lipid formulation containing the vaccine, which is stable when refrigerated or frozen, is then delivered to a vaccinee by either needle/syringe, jet injection, or microneedles. It had been found that by combining the nucleic acid vaccine with the lipid formulation, the level of immunogen, or immune response molecules, produced *in vivo* is increased and, in the case of a vaccine immunogen, the immune response is enhanced.

### BACKGROUND OF THE INVENTION

Numerous nucleic acid vaccines for the protection and/or treatment of human and animal diseases (e.g. infectious diseases and cancer) are presently in clinical trials. In addition, nucleic acid vaccines are currently being used to produce candidate monoclonal and polyclonal antibody products for use as therapeutics, prophylactics, and assay reagents. One of the acknowledged drawbacks to nucleic acid vaccines is the lack of sufficient potency of the vaccines, especially for the production of immune responses in humans.

In particular, DNA vaccines are currently being used to produce candidate monoclonal and polyclonal antibody products for use as therapeutics, prophylactics, and assay reagents. DNA vaccination involves the direct introduction into appropriate tissues of a plasmid containing the DNA sequence encoding the antigen(s) against which an immune response is sought. One drawback of DNA vaccines in particular is the potency of the vaccines, especially for the production of immune responses in humans. Methods to enhance potency include adjuvants, and using delivery technology that increases delivery of the DNA vaccine plasmid to cells (for example electroporation and jet injection). However, no method or composition has yet been found that produces exceptionally high levels of immunogen, i.e., potency.

In view of the lack of potency of nucleic acid vaccines, in particular DNA vaccines, using traditional delivery methods, a method of enhancing the potency of plasmid-based nucleic acid vaccines and immunotherapies is desired.

WO 2017/019523 discloses methods for preventing, ameliorating or treating a disease associated with glioma in a subject in need, the method comprising administering to the subject an effective amount of a nanoparticle composition comprising micro RNA.

### SUMMARY OF THE INVENTION

The present invention provides a novel plasmid-based DNA vaccine composition comprising a lipid formulation, and vaccine immunogens or immune response molecules. In addition, the present invention provides a method of formulating a plasmid-based DNA vaccine, the method comprising formulating a plasmid-based DNA vaccine in a lipid formulation. The present invention increases vaccine potency dramatically. It was unexpectedly discovered that the level of immunogen, or immune response molecules, produced *in vivo* is increased (versus administering merely the vaccine or immunotherapy) and, in the case of a vaccine immunogen, the immune response is enhanced.

In particular, a purified plasmid DNA encoding vaccine immunogens, or immune response molecules, is combined in a lipid formulation which is stable when refrigerated or stored frozen. The DNA vaccine and lipid formulation is then delivered to a vaccinee by either needle/syringe, jet injection, or microneedles. The lipid formulation contains one or more symmetric ionizable cationic lipids selected from among the following:

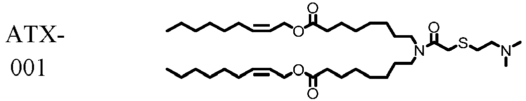

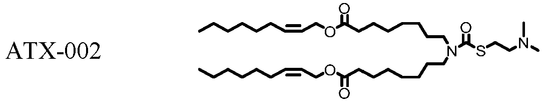

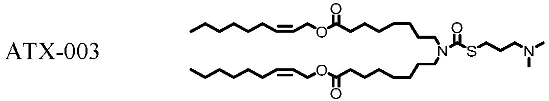

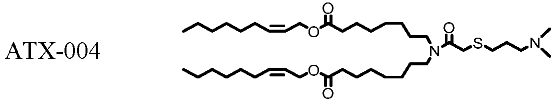

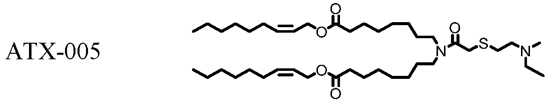

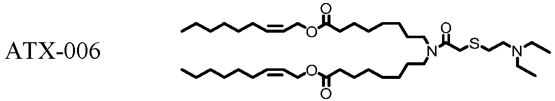

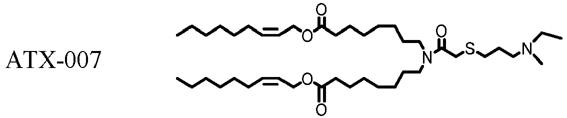

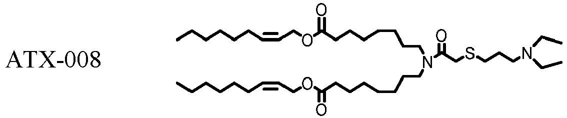

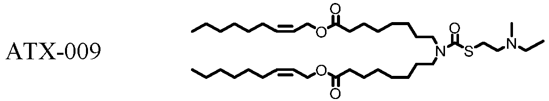

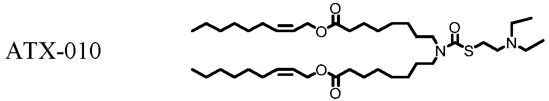

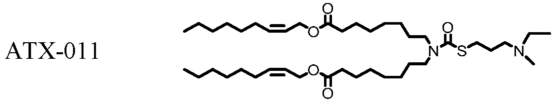

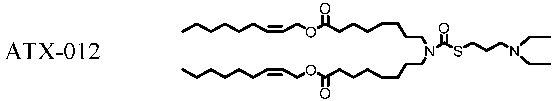

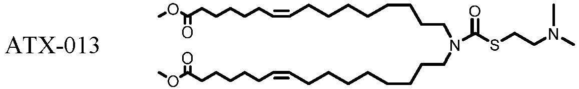

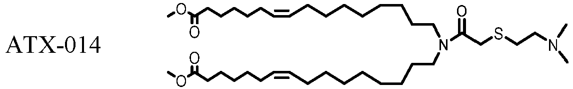

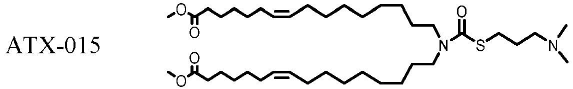

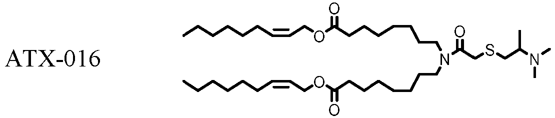

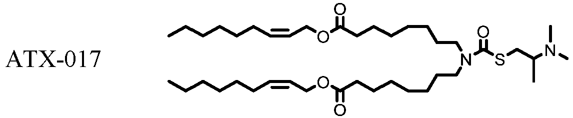

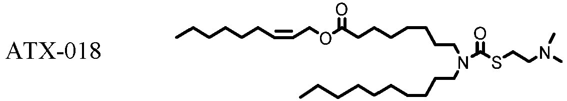

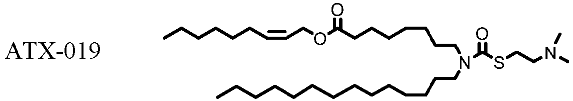

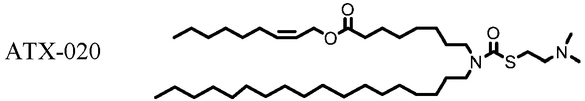

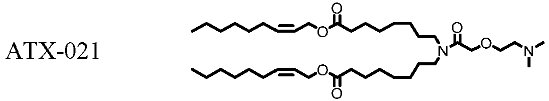

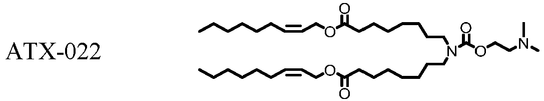

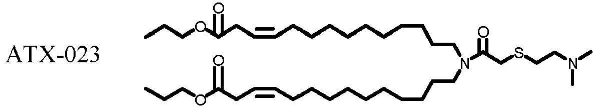

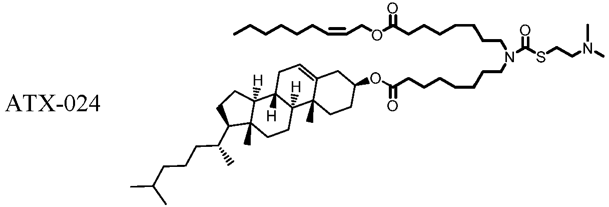

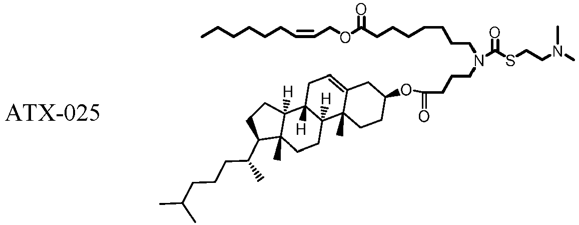

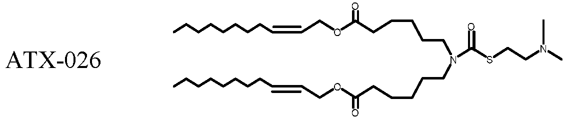

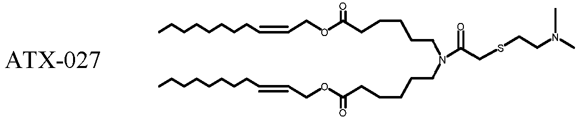

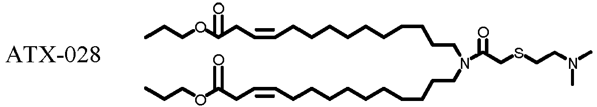

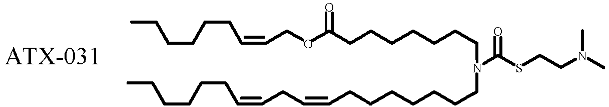

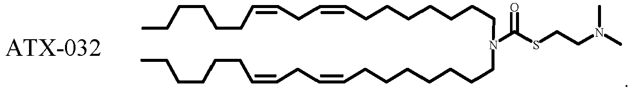

In a further embodiment, the lipid formulation mentioned above further contains one or more lipid excipients selected from 1,2-Distearoyl-sn-glycero-3-phosphocholine, Cholest-5-en-3β-ol, 1,2-Dimyristoyl-rac-glycero-3-methylpolyoxyethlyene.

### BRIEF DESCRIPTION OF THE SEVERAL VIEWS OF THE DRAWINGS

Figure 1 is a graph illustrating the blood concentrations (in ng/ml) of human monoclonal antibodies detected and quantified in a rabbit at various time periods after administration of lipid-formulated DNA plasmid encoding the monoclonal antibody heavy(H) and light(L) chains, and controls thereto.
Figure 2A is a graph illustrating the Andes virus pseudovirion neutralization levels of the rabbits mentioned above, over a period of 28 days.
Figure 2B is a graph illustrating the Andes virus plaque reduction neutralization test levels (PRNT) of the rabbits mentioned above, over a period of 28 days.
Figure 3A is a graph illustrating the results of a further plaque reduction neutralization test (PRNT) conducted with the two rabbits mentioned above, wherein the mean % neutralization vs. reciprocal sera dilutions from the two independent experiments was plotted is shown
Figure 4A shows an overview of an experiment comparing the immunogenicity of formulated versus unformulated Andes DNA vaccine in rabbits. Figure 4B shows individual rabbit neutralizing antibody responses. Neutralizing antibodies were measured by Andes virus pseudovirion neutralization assay. 50% neutralization titers (PsVNA50) are reported. Figure 4C shows a grouped neutralizing response. The Table in Figure 4 contains the mean PsVNA50 titers and percent coefficient of variation (%CV) per day between the two groups that received either formulated or unformulated Andes DNA vaccine.

### DETAILED DESCRIPTION OF THE INVENTION

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used.

It is also to be understood that the terminology used herein is for describing particular embodiments only and is not intended to be limiting.

The articles "a" and "an" are used herein to refer to one or to more than one (i.e., to at least one) of the grammatical object of the article. By way of example, "an element" means one element or more than one element. Thus, recitation of "a cell", for example, includes a plurality of the cells of the same type.

As discussed above, the present invention provides a novel nucleic acid vaccine composition comprising a lipid formulation, and vaccine immunogens or immune response molecules, wherein the lipid formulation is comprised of a symmetric ionizable cationic lipid.

The nucleic acid vaccine is a DNA vaccine, in particular a purified plasmid DNA encoding vaccine immunogens, or immune response molecules. Such DNA vaccines can include, for example, hantavirus vaccines including those targeting Andes virus, Sin Nombre virus, Hantaan virus, and Puumala virus; South American arenavirus vaccines including those targeting Junin virus, Machup virus, Guanarito virus, and Sabia virus; poxvirus DNA vaccines, alphavirus DNA vaccines, filovirus DNA vaccines, and Zika virus DNA vaccines. The preceding list includes anti-viral DNA vaccines, but DNA vaccines against cancer and other infectious diseases are possible vaccines-of-interest.

Hentavirus vaccines are described, for example, in Hooper JW, R. L. Brocato, S. A. Kwilas, CD Hammerbeck, MD Josleyn, M Royals, J Ballantyne, H Wu, J Jiao, H Matsushita, and E. J. Sullivan. DNA vaccine-derived human IgG produced in transchromosomal bovines protect in lethal models of hantavirus pulmonary syndrome. Sci Transl Med. 2014 Nov 26;6(264):264ra162. Kwilas S, Kishimori J, Josleyn M, Jerke K, Ballantyne J, Royals M, Hooper JW (2014).A Hantavirus Pulmonary Syndrome (HPS) DNA Vaccine Delivered using a Spring-Powered Jet Injector Elicits a Potent Neutralizing Antibody Response in Rabbits and Nonhuman Primates. Curr Gene Ther. Vol. 14, No. 3, 2014. Hooper JW, Josleyn MJ, Ballantyne J, and RL Brocato (2013) A novel Sin Nombre virus DNA vaccine and its inclusion in a candidate pan-hantavirus vaccine against hantavirus pulmonary syndrome (HPS) and hemorrhagic fever with renal syndrome (HFRS). Vaccine. 31(40):43314-21. Hooper JW, Moon J, Paolino K, Necomber R, McLain D, Josleyn MJ, Hannaman D, and CS Schmaljohn (2014). A Phase 1 clinical trial of Hantaan virus and Puumala virus M-segment DNA vaccines for hemorrhagic fever with renal syndrome delivered by intramuscular electroporation. Clinical Microbiology and Infection. May;20 Suppl 5:110-7. doi: 10.1111/1469-0691.12553. Epub 2014 Mar; Boudreau EF, Josleyn M, Ullman D, Fisher D, Dalrymple L, Sellers-Myers K, Loudon P, Rusnak J, Rivard R, Schmaljohn C, Hooper JW (2012). A Phase 1 clinical trial of Hantaan virus and Puumala virus M-segment DNA vaccines for hemorrhagic fever with renal syndrome. Vaccine 30:1951-1958.

Arenavirus DNA vaccines are described, for example, in Golden JW, M Zaitseva, S Kapnick, RW Fisher, MG Mikolajczyk, J Ballantyne, H Golding and JW Hooper (2011). Polyclonal antibody cocktails generated using DNA vaccine technology protect in murine models of orthopoxvirus disease. Virology Journal 2011, 8:441.

Alphavirus DVA vaccines are described, for example, in Golden JW, M Zaitseva, S Kapnick, RW Fisher, MG Mikolajczyk, J Ballantyne, H Golding and JW Hooper (2011). Polyclonal antibody cocktails generated using DNA vaccine technology protect in murine models of orthopoxvirus disease. Virology Journal 2011, 8:441.

Filovirus DNA vaccines are described, for example, in Bounds CE, Kwilas SA, Kuehne AI, Brannan JM, Bakken RR, Dye JM, Hooper JW, Dupuy LC, Ellefsen B, Hannaman D, Wu H, Jiao JA, Sullivan EJ, Schmaljohn CS (2015). Human Polyclonal Antibodies Produced through DNA Vaccination of Transchromosomal Cattle Provide Mice with Post-Exposure Protection against Lethal Zaire and Sudan Ebolaviruses. PLoS One sep 30;10(9):e0137786. Grant-Klein, RJ, Altamura LA, Badger CV, Bounds CE, Van Deusen NM, Kwilas SA, Vu HA, Warfield KL, Hooper JW, Hannaman D, Dupuy LC, and CS Schmaljohn (2015) Codon-Optimized Filovirus DNA Vaccines Delivered by Intramuscular Electroporation Protect Cynomolgus Macaques from Lethal Ebola and Marburg Virus Challenges. Human Vaccine and Immunotherapy. 2015 May 21:0.

Poxvirus vaccines are describd, for example, in Hooper, J. W., E. Thompson, C. Wilhelmsen, M. Zimmerman, M. Ait Ichou, S. E. Steffen, C. S. Schmaljohn, A. L. Schmaljohn, and P. B. Jahrling (2004). Smallpox DNA vaccine protects nonhuman primates against lethal monkeypox. Journal of Virology 78:4833-4843.

Zika DNA vaccines are described, for example, in Stein DR, Golden JW, Griffin BD, Warner BM, Ranadheera C, Scharikow L, Sloan A, Frost KL, Kobasa D, Booth SA, Josleyn M, Ballantyne J, Sullivan E, Jiao JA, Wu H, Wang Z, Hooper JW, Safronetz D (2017) Human polyclonal antibodies produced in transchromosomal cattle prevent lethal Zika virus infection and testicular atrophy in mice. Antiviral Research. 2017 Oct;146:164-173. Gaudinski MR, Houser KV, Morabito KM, Hu Z, Yamshchikov G, Rothwell RS, Berkowitz N, Mendoza F, Saunders JG, Novik L, Hendel CS, Holman LA, Gordon IJ, Cox JH, Edupuganti S, McArthur MA, Rouphael NG, Lyke KE, Cummings GE, Sitar S, Bailer RT, Foreman BM, Burgomaster K, Pelc RS, Gordon DN, DeMaso CR, Dowd KA, Laurencot C, Schwartz RM, Mascola JR, Graham BS, Pierson TC, Ledgerwood JE, Chen GL; VRC 319; VRC 320 study teams. Safety, tolerability, and immunogenicity of two Zika virus DNA vaccine candidates in healthy adults: randomised, open-label, phase 1 clinical trials.Lancet. 2017 Dec 4. pii: S0140-6736(17)33105-7. doi: 10.1016/S0140-6736(17)33105-7.

The skilled artisan will understand that the instantly described invention is not limited to these specific DNA vaccines, but may also be used with any other DNA vaccines known to the skilled artisan.

The symmetric ionizable cationic lipid is selected from among:

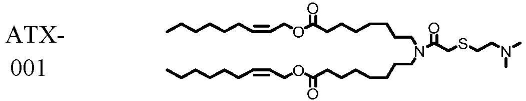

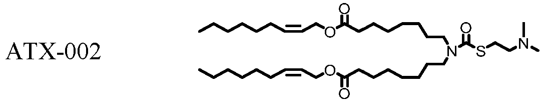

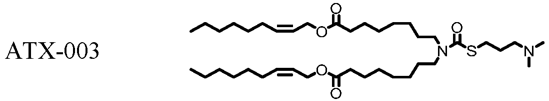

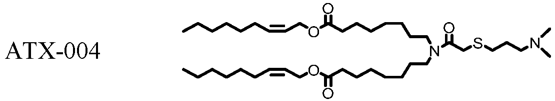

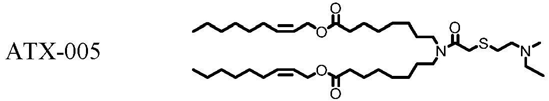

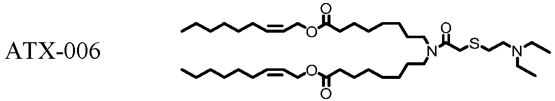

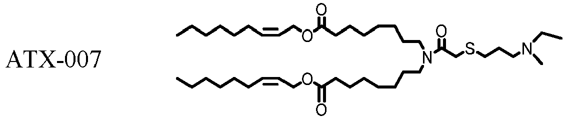

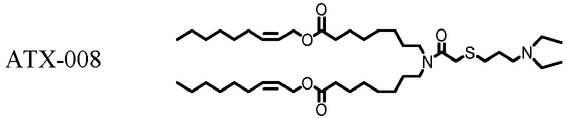

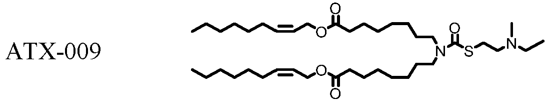

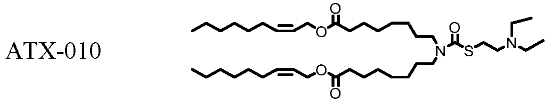

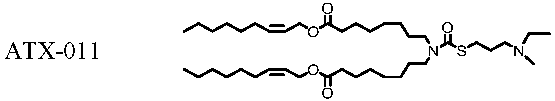

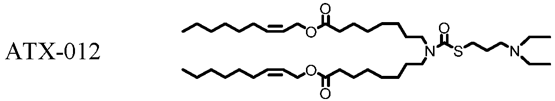

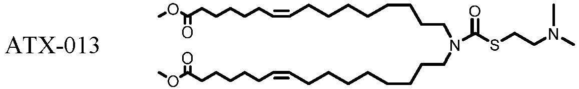

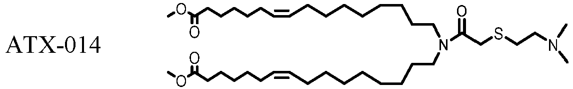

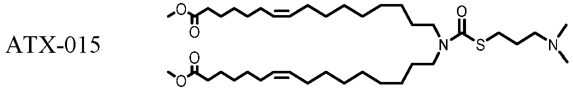

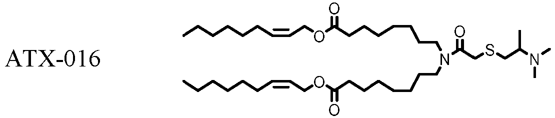

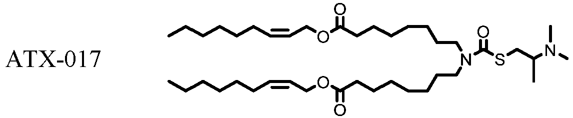

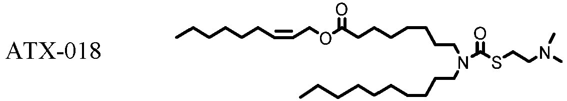

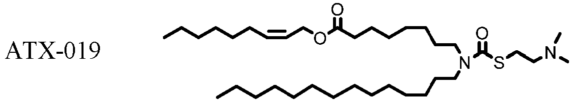

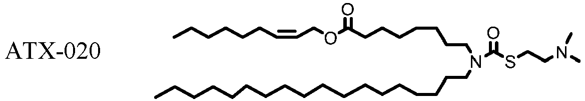

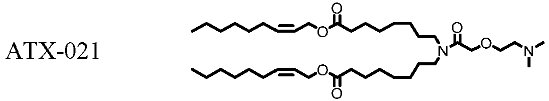

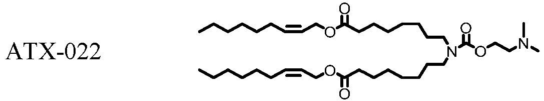

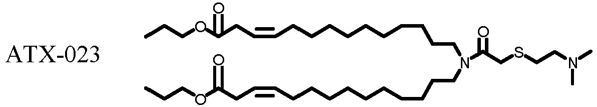

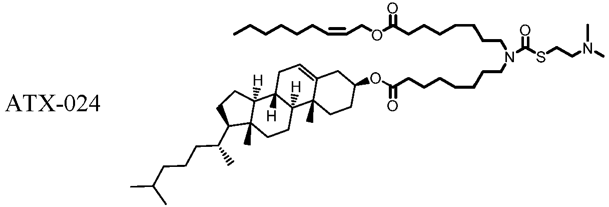

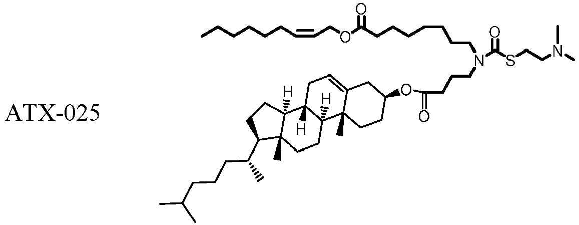

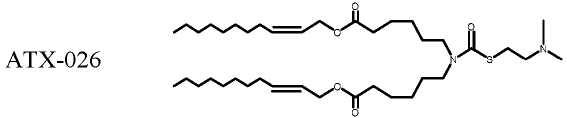

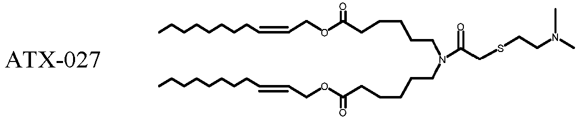

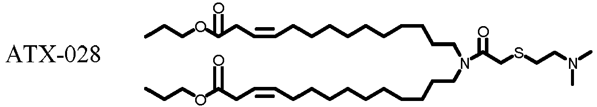

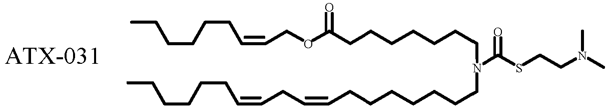

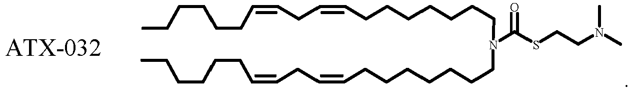

What are also described herein are any of the compounds listed in ATX-001 to ATX-032, or a pharmaceutically acceptable salt thereof, in a lipid composition, comprising a nanoparticle or a bilayer of lipid molecules. The lipid bilayer preferably further comprises a neutral lipid or a polymer. The lipid composition preferably comprises a liquid medium. The composition preferably further encapsulates a nucleic acid, which is a DNA vaccine. The lipid composition preferably further comprises a nucleic acid and a neutral lipid or a polymer. The lipid composition preferably encapsulates the nucleic acid.

These symmetric ionizable cationic lipids can exist in unsolvated and solvated forms, including hydrated forms. In general, the solvated forms, with pharmaceutically acceptable solvents such as water, ethanol, and the like, are equivalent to the unsolvated forms for the purposes of this disclosure. These symmetric ionizable cationic lipids and salts, solvates thereof, may exist in their tautomeric form (for example, as an amide or imino ether). All such tautomeric forms are contemplated herein as part of the present disclosure. These symmetric ionizable cationic lipids, and their methods of synthesis, are described for example in U.S. 9,593,077. The synthetic methods set forth in that reference for the cationic lipid compounds can be synthesized with the skills in the art. The skilled of the art will recognize other methods to produce these compounds. For Example, ATX-001 can be made, for example, by the following procedure:
Di((Z)-non-2-en-1-yl) 8,8'((tertbutoxycarbonyl)azanediyl) dioctanoate (0.023 mol, 15 g) was dissolved in dry dichloromethane (DCM) (200 ml). Trifluoroacetic acid (TFA) was added at 0° C. to initiate a reaction. The reaction temperature was slowly allowed to warm to room temperature over for 30 minutes with stirring. Thin layer chromatography showed that the reaction was completed. The reaction product was concentrated under vacuum at 40° C. and the crude residue was diluted with DCM, and washed with a 10% NaHCO₃ solution. The aqueous layer was re-extracted with DCM, and the combined organic layers were washed with brine solution, dried over Na₂SO₄ and concentrated. The collected crude product (12 grams) was dissolved in dry DCM (85 ml) under nitrogen gas. Triphosgene were added and the reaction mixture was cooled to 0° C., and Et₃N was added drop wise. The reaction mixture was stirred overnight at room temperature. Thin layer chromatography showed that the reaction was completed. DCM solvent was removed from the reaction mass by distillation under N₂. The reaction product was cooled to 0° C., diluted with DCM (50 ml), and 2-((2-(dimethylamino)ethyl)thio) acetic acid (0.039 mol, 6.4 g) and carbodiimide (EDC HCl) (0.054 mol, 10.4 g). The reaction mixture was then stirred overnight at room temperature. Thin layer chromatography showed that the reaction was completed. The reaction product was diluted with 0.3M HCl solution (75 ml), and the organic layer was separated. The aqueous layer was re-extracted with DCM, and the combined organic layers were washed with 10% K₂CO₃ aqueous solution (75 ml) and dried over anhydrous Na₂SO₄. Concentration of the solvent gave a crude mass of 10 gram. The crude compound was purified by silica gel column (100-200 mesh) using 3% MeOH/DCM. The yield was 10.5 g (68%).

The cationic lipid compounds may be combined with an agent to form microparticles, nanoparticles, liposomes, or micelles. The agent to be delivered by the particles, liposomes, or micelles may be in the form of a gas, liquid, or solid, and the agent may be a polynucleotide, protein, peptide, or small molecule. The lipomacrocycle compounds may be combined with other cationic lipid compounds, polymers (synthetic or natural), surfactants, cholesterol, carbohydrates, proteins, lipids, etc. to form the particles. These particles may then optionally be combined with a pharmaceutical excipient to form a pharmaceutical composition.

The present description provides novel DNA vaccines based on the use of such cationic lipid compounds. The system may be used in the pharmaceutical/drug delivery arts to deliver polynucleotides, proteins, small molecules, peptides, antigen, drugs, etc. to a patient, tissue, organ, or cell. These novel compounds may also be used as materials for coating, additives, excipients, materials, or bioengineering
The lipids used in the instant invention can, for example, be in the form of particles. Such lipid particles may have, for example, a mean diameter of approximately 80-110 nm with low polydispersity values (< 0.2). In one embodiment, the lipid of the present invention is an ionizable lipid that is positively charged at low pH but relatively neutral at physiological pH.

The nucleic acid vaccine is formulated in the lipid formulation, for example, by encapsulating the DNA with the lipid formulation disclosed herein. As a result, the lipid formulation encapsulates and protects the immunogen/immune response molecules in nano-scale lipid particles comprised of specialized lipid components. The DNA vaccine compositions of this disclosure may be administered by various routes, for example, to effect systemic delivery via intravenous, parenteral, intraperitoneal, or topical routes.

Functionally, the lipid is responsible for binding the nucleic acid and promoting endosomolytic activity. This delivery system is also optimized for high nucleic acid encapsulation efficiency (important for manufacturing/cost-of-goods) and exquisite size control (important for the regulatory authority approval). The lipid particles fuse to a cell membrane and deliver its nucleic acid payload to the cytoplasm by using a pH-mediated disruption of the endosome followed by rapid biodegradation of the lipid inside the cell.

In an additional embodiment, the lipid formulation utilized in the instant invention further contains one or more lipid excipients selected from 1,2-Distearoyl-sn-glycero-3-phosphocholine, Cholest-5-en-3β-ol, or 1,2-Dimyristoyl-rac-glycero-3-methylpolyoxyethlyene. These additional lipid excipients can be mixed with the symmetric ionizable cationic lipids described above to form the lipid formulation.

### EXAMPLE 1: VACCINATION WITH UNFORMULATED DNA VACCINE

In previous studies, rabbits were vaccinated with unformulated Andes DNA vaccine and the following results were obtained:
Study 1 (unformulated DNA). Eight rabbits were vaccinated with unformulated Andes DNA vaccine using PharmaJet IM, 2 mg/injection. After 1 vaccination (Week 4) PsVNA80= 81, 40, 57, <20, <20, <20, 43, 34. GMT= 27 (when <20 are given value of 10).
Study 2 (unformulated DNA). A preclinical toxicity study in rabbits was performed. Eight rabbits received 4 vaccinations using unformulated Andes DNA vaccine, 2 mg/injection x 4 injections per vaccination (a total of 32 mg of DNA). The PsVNA80 after 3 vaccinations was GMT=**7,781** (Lower 95% 5,061, Upper 95% 11,966) and after 4 vaccinations was **18,899** (Lower 95% CI 7,181, Upper 95% CI 49,738) (J. Hooper, Contributing Scientist Report AN-8327478-G, unpublished). This represents the predicted maximum neutralizing activity that can be produced in rabbits vaccinated with unformulated Andes DNA vaccine delivered by disposable syringe jet injection.

### EXAMPLE 2: VACCINATION WITH DNA VACCINE FORMULATED WITH THE INVENTIVE LIPID FORMULATION

The present inventors hypothesized that the lipid formulation disclosed herein may increase the potency of nucleic acid vaccines. To evaluate this possibility, testing was performed on a rabbit, wherein the rabbit was given a single intramuscular injection (PharmaJet Stratis) of a vaccine containing 1mg of DNA vaccine plasmid pWRG/c7d11 (H+L) combined with the lipid formulation of the present invention. Sera specimens were collected before injection (Day-4, relative to injection) and after (Days 1-5, 7, 10, 14 and 21, relative to injection). Recombinant monoclonal human antibody was detected and quantified from the sera by an immunogen specific ELISA. The data is presented with historical controls (n=4) in which rabbits received four intramuscular injections of plasmid pWRG/c7d11 (H+L) (1 mg/injection, 4 mg total).

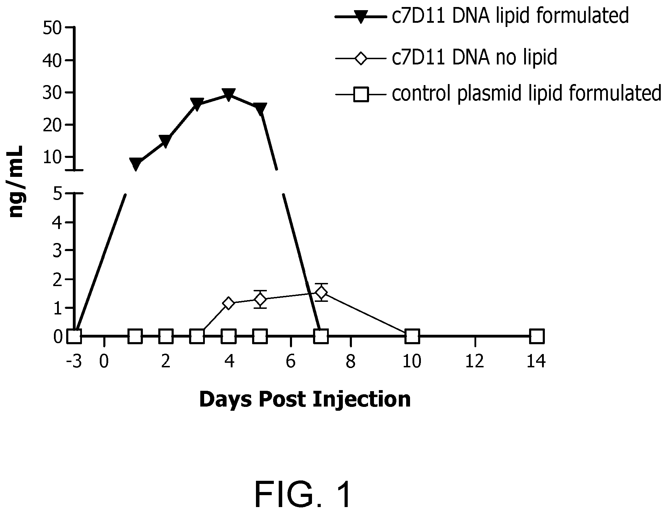

As shown in Figure 1 below, these tests demonstrated that the lipid formulation of a plasmid expressing a protein-of-interest resulted in a >10-fold increase in serum levels of that protein in injected rabbits while using one quarter of the amount of nucleic acid. In particular, human monoclonal antibody was detected and quantifiable 24 hours after injection of the lipid formulated plasmid, whereas unformulated plasmid nucleic acid was not detectable until at least day 5. Sera from rabbits injected with formulated nucleic acid yielded approximately 30 ng/ml. In contrast, sera from rabbits injected with unformulated (i.e., without being blended in a lipid formulated) nucleic acid ranged from approximately 0.4 ng/ml to 2/4 ng/ml, substantially less than the concentration obtained with the formulated nucleic acid.

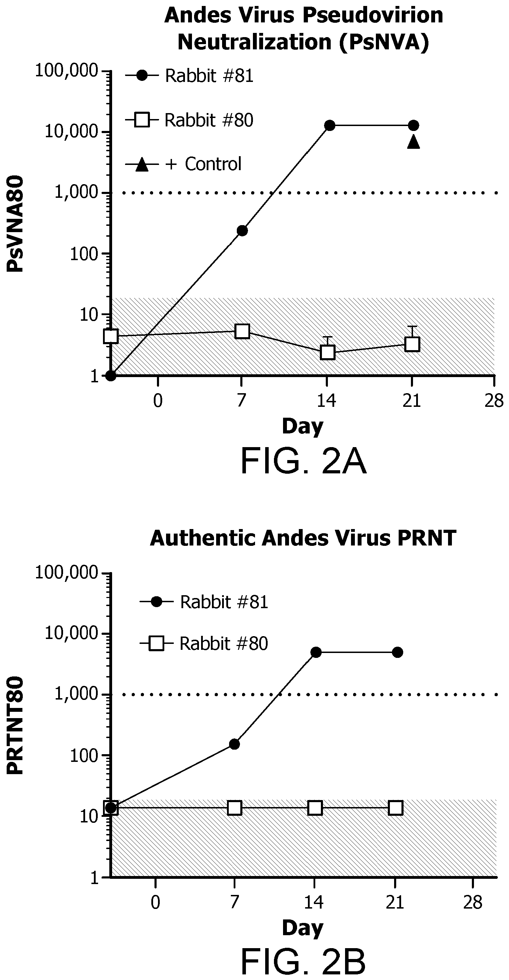

Next, a rabbit (designated as rabbit #81) was vaccinated using PharmaJet needle-free jet injection (intramuscular) on Day 0, relative to injection, with 1 mg of lipid-formulated Andes virus (ANDV) DNA vaccine. Rabbit #80 was vaccinated with the same lipid-formulated DNA vaccine vector as a control (i.e., pWRG/c7d11[H+L] above). Serum collected on Days -4-7, 14, and 21 were evaluated for neutralizing activity by pseudovirion neutralization assay (PsVNA) and by plaque reduction neutralization test (PRNT) using Andes virus. As shown in Figure 2A below, PsVNA80 titers were plotted. Each symbol represents the mean ±SD of duplicates. The positive control was Day 116 sera from a rabbit vaccinated 3x with the ANDV DNA vaccine by intramuscular electroporation. As shown in Figure 2B, PRNT80 titers were plotted. Each symbol represents the mean ±SD of two independent experiments. The limit of quantitation was 20 (shaded grey) for both PRNT and PsVNA. PsVNA80 and PRNT80 titers greater than 1,000 are considered high (dotted line).

The results of the above described tests indicated that neutralizing antibodies were detected as early as Day 7 after injection of the lipid-formulated ANDV DNA vaccine, and reached high titer by Day 14. The Day 14 and 21 PsVNA80 tites were as high or higher than those obtained historically using intramuscular electroporation (Hooper et al. Journal of Virology: Vo82 No. 3 p. 1331-1338. See figure 2 in that publication). The PRNT titers were similar to the PsVNA titers and demonstrated that the neutralizing antibody activity was against authentic virus. There was no neutralizing activity in the negative control rabbit as measured by PRNT. Mean Day 14 and 21 PsVNA80 titers were 14,150 and 14,507, respectively. Mean Day 14 and 21 PRNT80 titers were both 5120. PsVNA50 and PRNT50 titers were >10,000 after a single vaccination (data not shown). Accordingly, it was shown that use of lipid-formulated plasmid DNA vaccine increased the neutralizing antibody response dramatically, and increases immunogenicity of DNA vaccines.

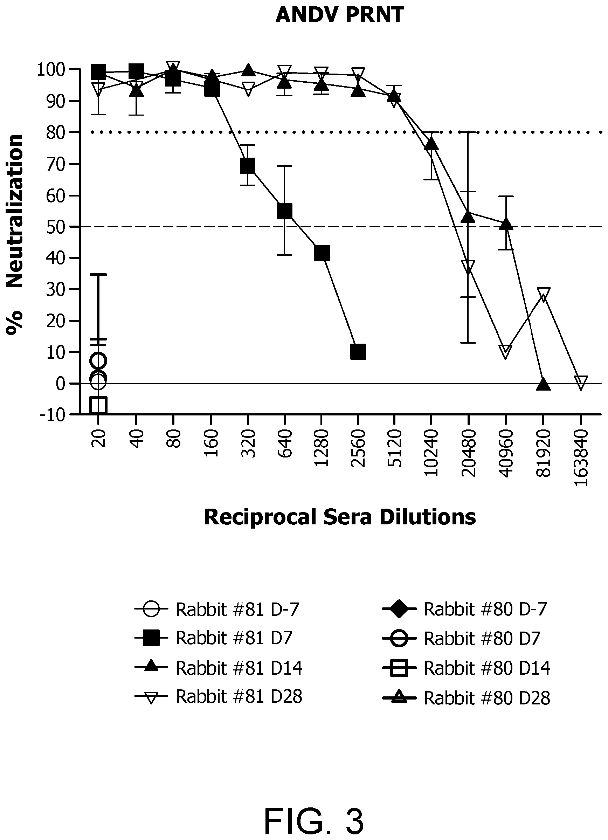

In a subsequent plaque reduction neutralization test (PRNT), rabbit #81 was administered a single injection of formulated ANDV DNA vaccine, pWRG/AND-M(opt2) on Day 0. Sera were collected on Days -7, 7, 14, and 28 and evaluated in an ANDVPRNT. The mean % neutralization from two independent experiments was plotted, and the results presented in Figure 3 below. It was found that rabbit #80 did not exhibit neutralizing activity at the lowest dilution tested (1:20) for any timepoint. Rabbit#81 did not exhibit neutralizing antibodies until after vaccination. At the first timepoint after vaccination (D7), Rabbit#81 already had substation levels of neutralizing antibodies in its serum (PRNT80=160). By D14 this animal had very high levels of neutralizing activity (PRNT80=5120). The PRNT80 titer was the same on D28. PRNT80 is defined as the reciprocal of the highest dilution that reduces plaque number by 80% relative to No Antibody control wells. The test data demonstrated that a rabbit vaccinated with a lipid formulated ANDV DNA vaccine (i.e., a formulation containing the lipid of the present invention) developed high-titer neutralizing antibodies against authentic Andes virus (ANDV). Note that the PRNT titer data is plotted in Fig. 2

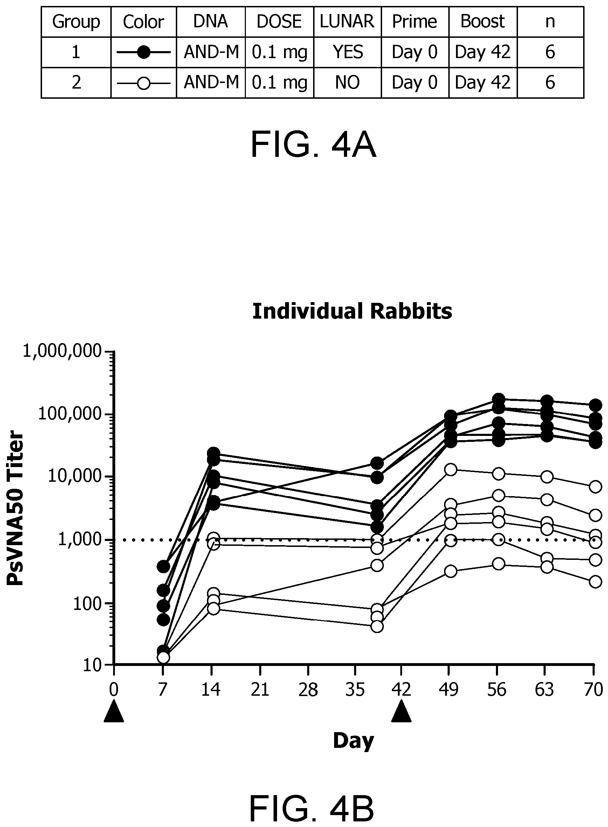

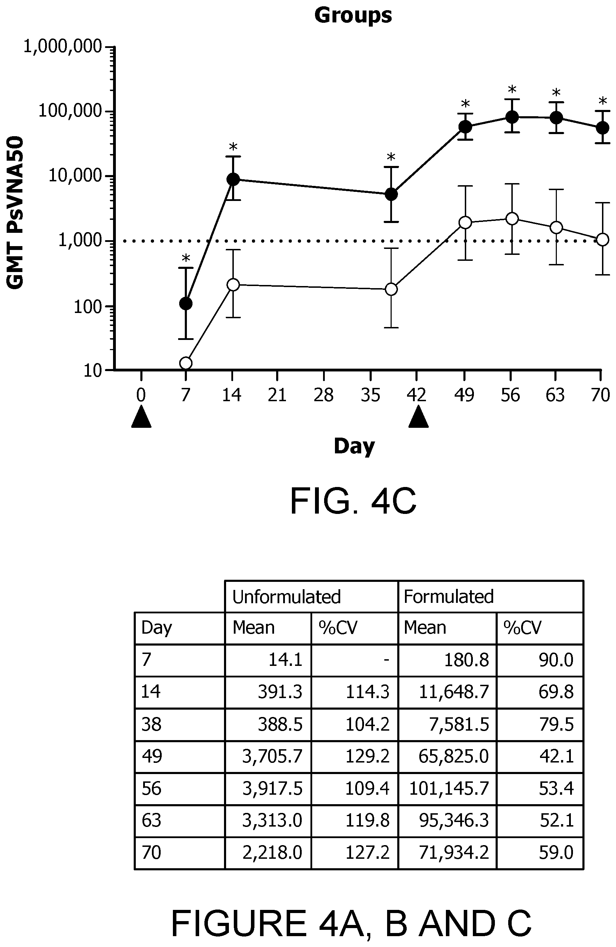

In another study, the effect of formulations on the neutralizing responses produced by rabbits vaccinated with ANDV DNA was compared. Utilizing twelve rabbits, 6 female rabbits per group were injected with either the formulated ANDV DNA vaccine or the DNA vaccine in the absence of formulation (unformulated) (Fig 4A). All the animals received the same concentration of DNA (100 ug). A vaccine boost was administered 42 days later. From the sera of these rabbits, neutralizing responses were measured using the PsVNA. Animals that received the formulated vaccine had detectable neutralizing activity 7 days after vaccination (PsVNA50 mean of 180.8), whereas animals in the unformulated did not (background of 14.1) (Fig. 4 table). At all time-points analyzed, sera from the formulated group had a higher PsVNA50 (Fig. 4 B, C). With the exception of Day 14, PsVNA50 values were at least 10 times larger from sera of the animals receiving formulated vaccine than to animals receiving the unformulated DNA. This number difference increased to 18-32 times following the second vaccination (boost) (Fig 4 Table). Also, PsVNA 50 neutralizing responses varied less between the animals vaccinated with the formulated DNA than between the unformulated DNA group. That is, the coefficient of variation was consistently less in the formulated ANDV DNA group at all time points.

## Claims

1. A plasmid-based DNA vaccine composition comprising a lipid formulation, and vaccine immunogens or immune response molecules, wherein the lipid formulation comprises at least one lipid selected from the group consisting of:

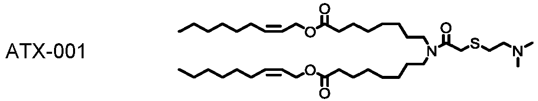

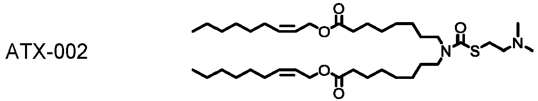

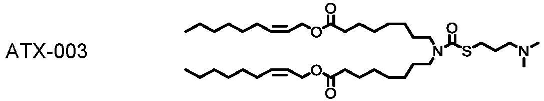

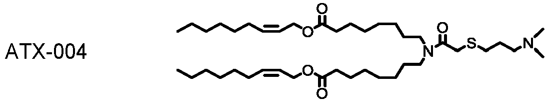

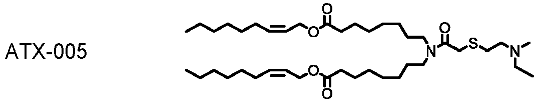

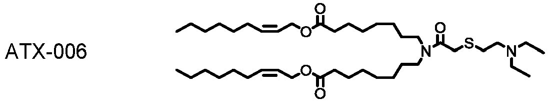

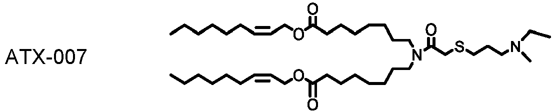

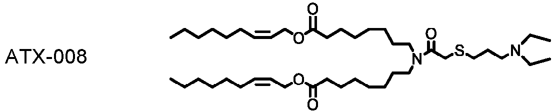

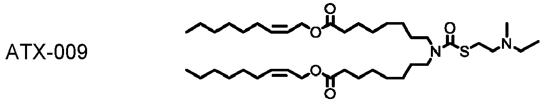

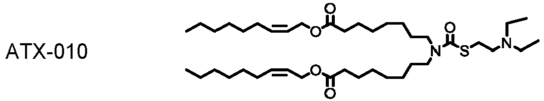

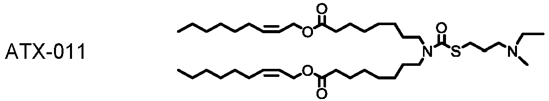

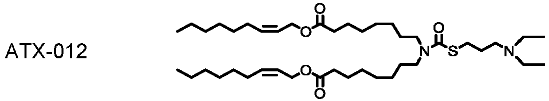

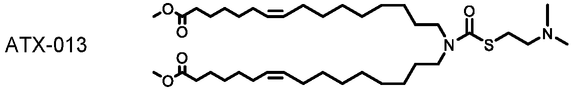

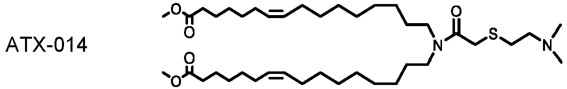

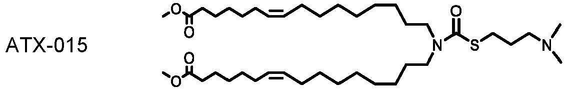

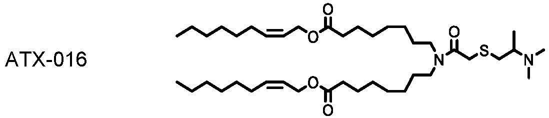

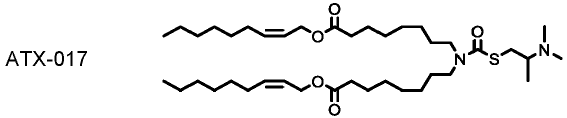

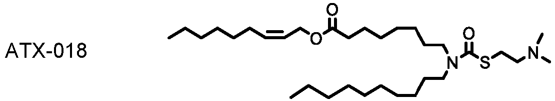

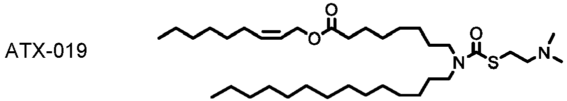

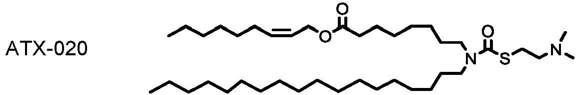

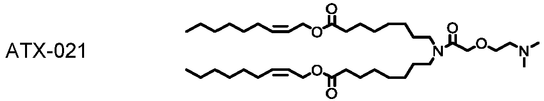

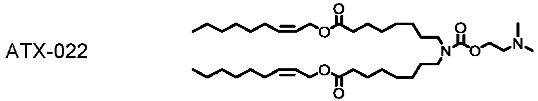

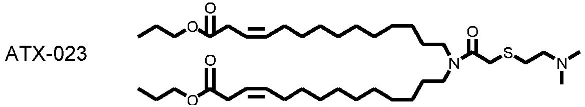

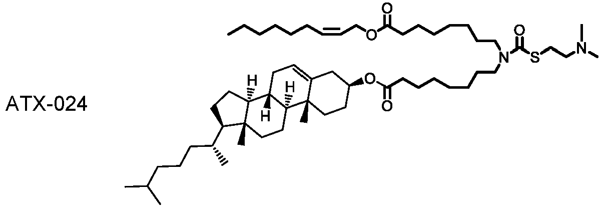

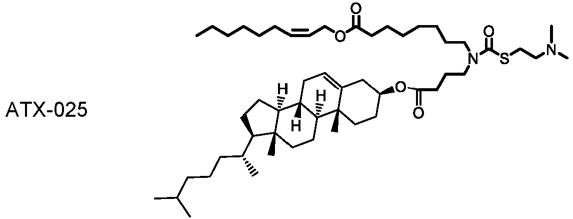

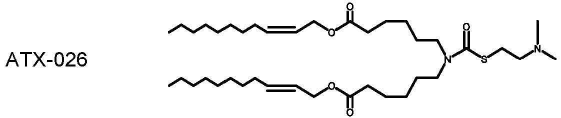

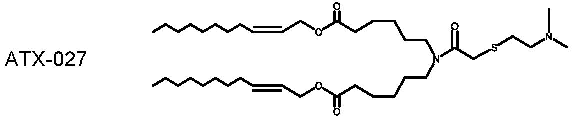

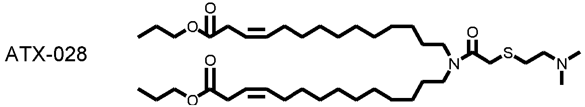

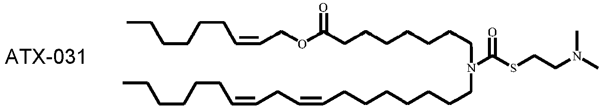

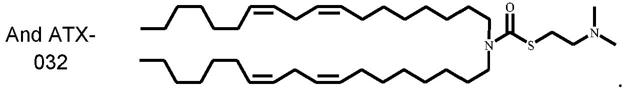

2. The plasmid-based DNA vaccine composition of claim 1, wherein the lipid formulation further comprises one or more lipid excipients selected from the group consisting of 1,2-Distearoyl-sn-glycero-3-phosphocholine, Cholest-5-en-3β-ol, and 1,2-Dimyristoyl-rac-glycero-3-methylpolyoxyethlyene.

3. The plasmid-based DNA vaccine composition of claim 1or 2, wherein the plasmid-based DNA vaccine is selected from the group consisting of hantavirus vaccines including those targeting Andes virus, Sin Nombre virus, Hantaan virus, and Puumala virus; South American arenavirus vaccines including those targeting Junin virus, Machup virus, Guanarito virus, and Sabia virus; poxvirus DNA vaccines, alphavirus DNA vaccines, filovirus DNA vaccines, and Zika virus DNA vaccines.

4. The plasmid-based DNA vaccine composition of any one of claims 1 to 3, wherein the plasmid-based DNA vaccine composition is stable when refrigerated or when stored frozen.

5. The plasmid-based DNA vaccine composition of any one of claims 1 to 4, wherein the lipids are in the form of particles.

6. The plasmid-based DNA vaccine composition of claim 5, wherein the particles of the lipid formulation have a mean diameter of 80-110 nm and a polydispersity of less than 0.2.

7. The plasmid-based DNA vaccine composition of any one of claims 1 to 6, wherein the plasmid-based DNA vaccine is encapsulated with the lipid formulation.

8. A method of formulating a plasmid-based DNA vaccine the method comprising formulating a plasmid-based DNA vaccine in a lipid formulation comprising a lipid selected from the group consisting of:

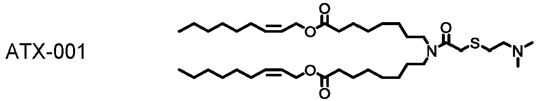

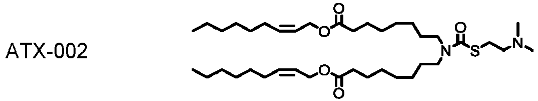

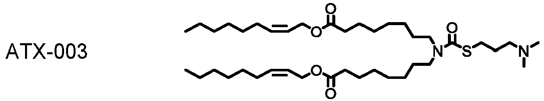

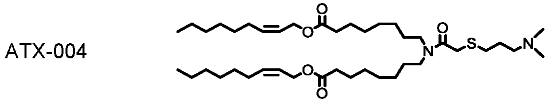

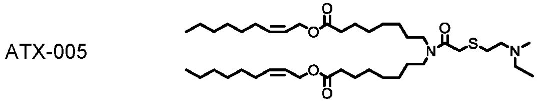

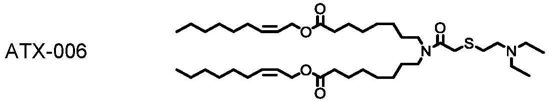

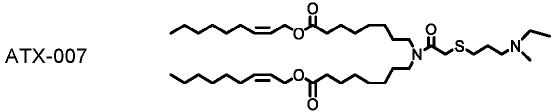

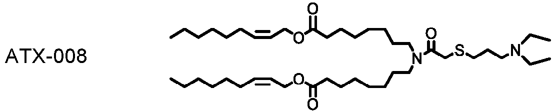

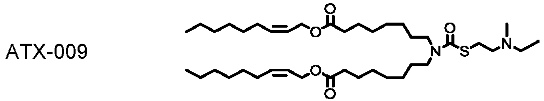

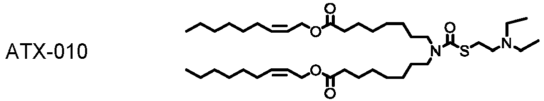

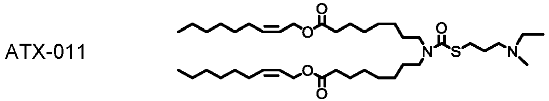

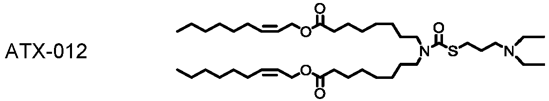

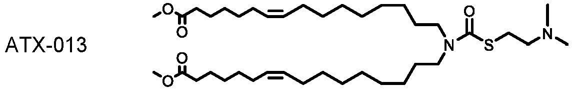

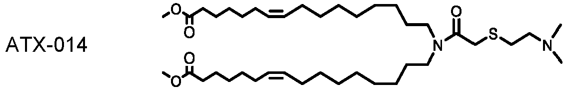

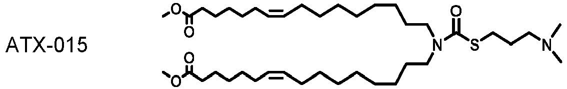

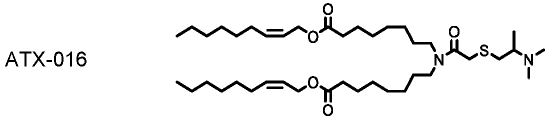

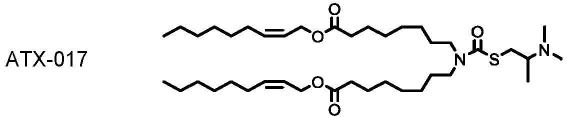

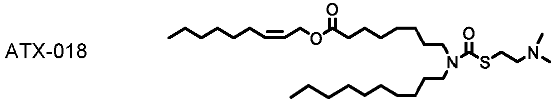

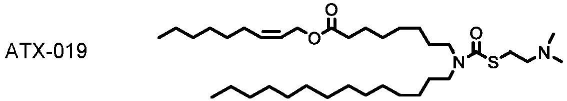

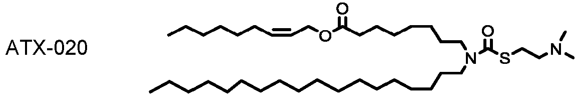

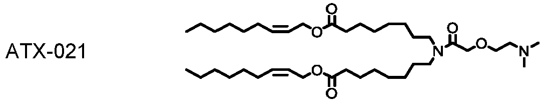

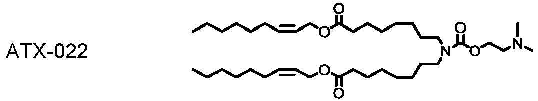

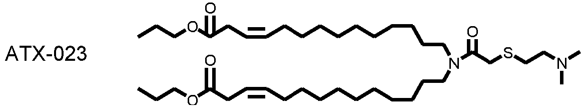

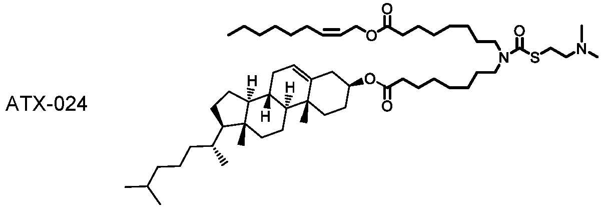

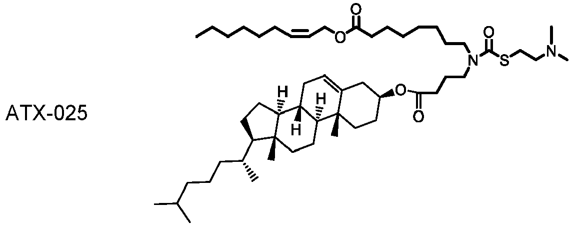

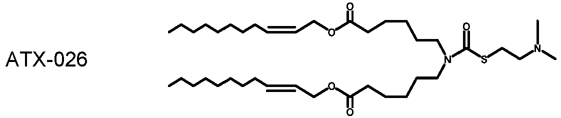

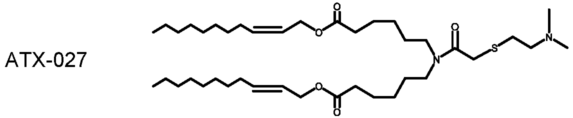

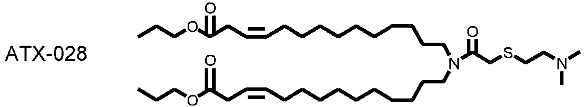

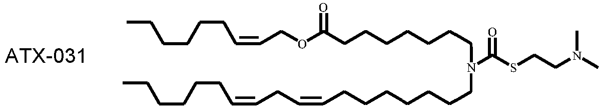

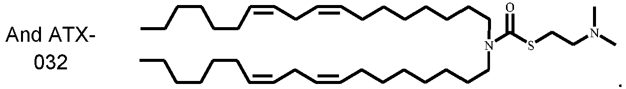

9. The method of claim 8, wherein the lipid formulation further comprises one or more lipid excipients selected from the group consisting of 1,2-Distearoyl-sn-glycero-3-phosphocholine, Cholest-5-en-3β-ol, and 1,2-Dimyristoyl-rac-glycero-3-methylpolyoxyethlyene.

10. The method of claim 8 or 9, wherein the plasmid-based DNA vaccine is selected from the group consisting of hantavirus vaccines including those targeting Andes virus, Sin Nombre virus, Hantaan virus, and Puumala virus; South American arenavirus vaccines including those targeting Junin virus, Machup virus, Guanarito virus, and Sabia virus; poxvirus DNA vaccines, alphavirus DNA vaccines, filovirus DNA vaccines, and Zika virus DNA vaccines.

11. The method of any one of claims 8 to 10, wherein the lipids are in the form of particles.

12. The method of claim 11, wherein the particles of the lipid formulation have a mean diameter of 80-110 nm and a polydispersity of less than 0.2.

13. The method of any one of claims 8 to 12, wherein the plasmid-based DNA vaccine is encapsulated with the lipid formulation.

## Patentansprüche

1. Plasmidbasierte DNA-Impfstoffzusammensetzung, die eine Lipidformulierung umfasst sowie Impfstoff-Immunogene oder Immunantwortmoleküle, wobei die Lipidformulierung mindestens ein Lipid umfasst, ausgewählt aus der Gruppe bestehend aus

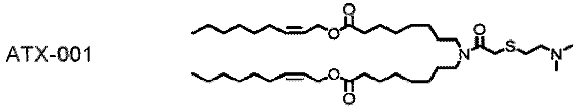

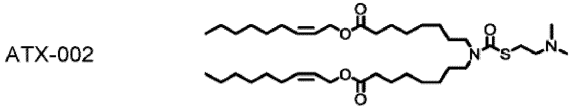

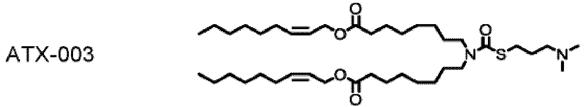

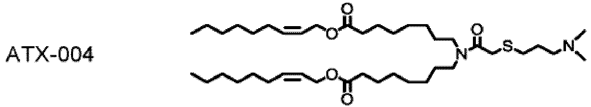

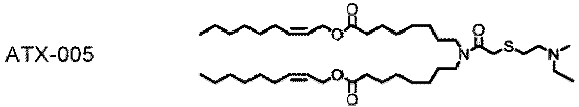

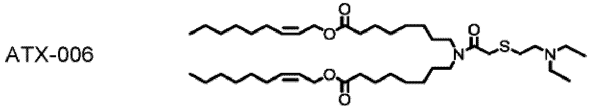

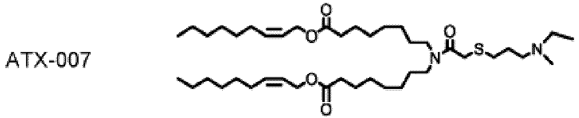

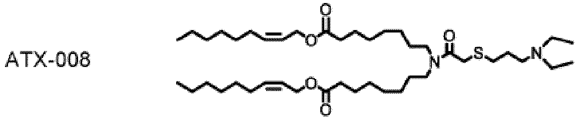

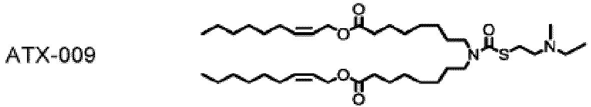

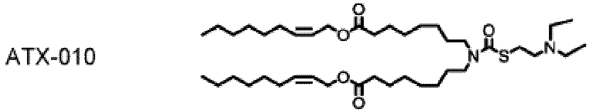

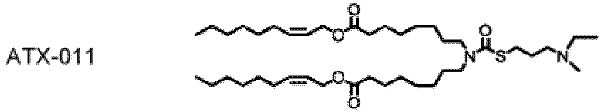

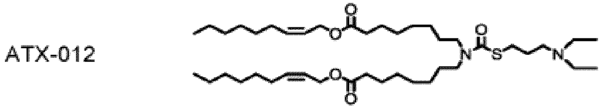

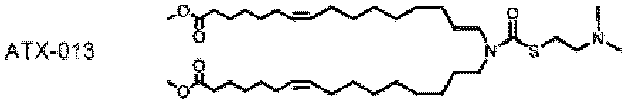

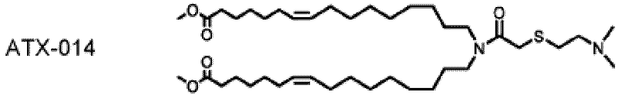

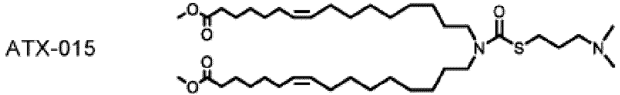

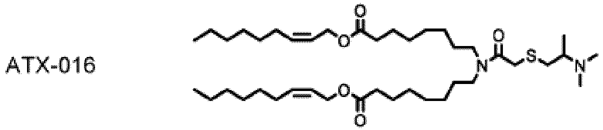

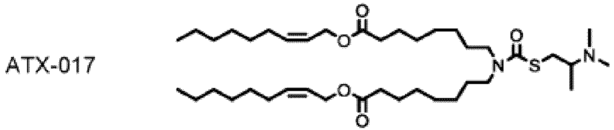

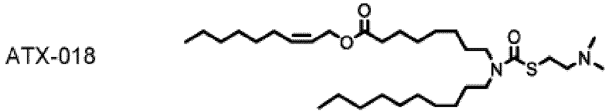

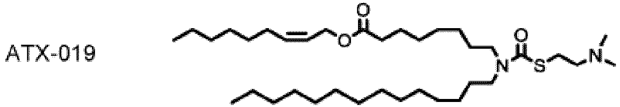

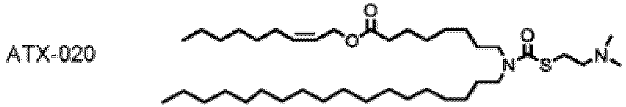

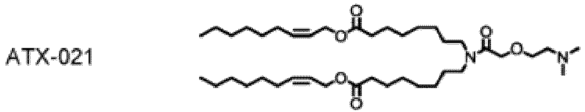

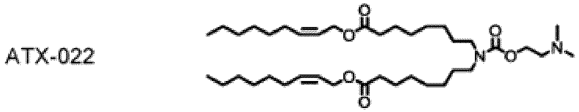

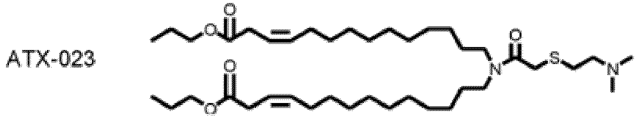

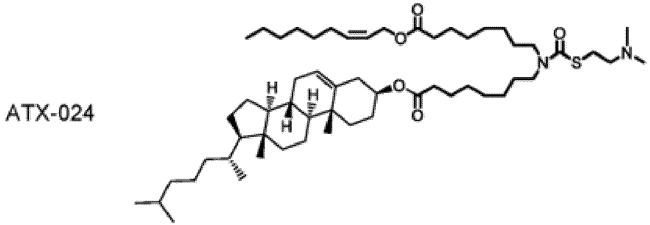

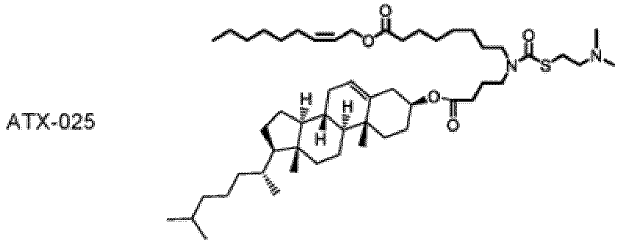

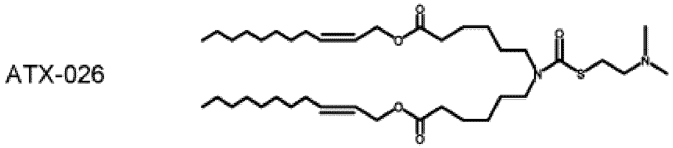

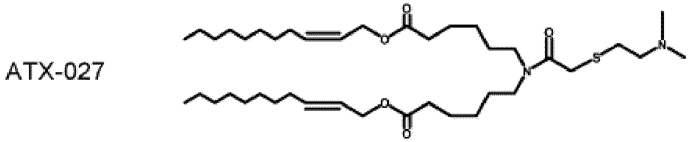

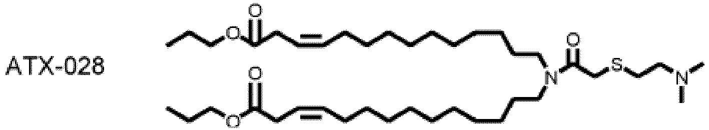

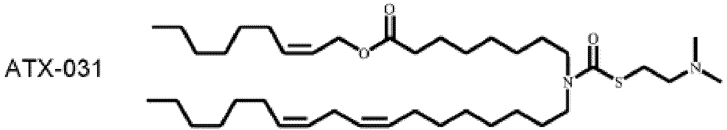

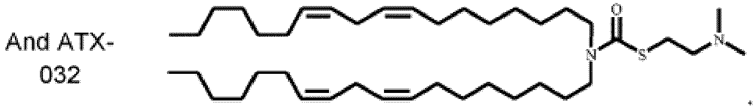

2. Plasmidbasierte DNA-Impfstoffzusammensetzung nach Anspruch 1, wobei die Lipidformulierung ferner einen oder mehrere Lipidhilfsstoffe umfasst, ausgewählt aus der Gruppe bestehend aus 1,2-Distearoyl-sn-glycero-3-phosphocholin, Cholest-5-en-3β-ol und 1,2-Dimyristoyl-rac-glycero-3-methyl-Polyoxyethylen.

3. Plasmidbasierte DNA-Impfstoffzusammensetzung nach Anspruch 1 oder 2, wobei der plasmidbasierte DNA-Impfstoff ausgewählt ist aus der Gruppe bestehend aus Hantavirus-Impfstoffen, die das Andes-Virus, das Sin-Nombre-Virus, das Hantaan-Virus und das Puumala-Virus enthalten; südamerikanische Arenavirus-Impfstoffe, die solche, die gegen das Junin-Virus, das Machup-Virus, das Guanarito-Virus und das Sabia-Virus gerichtet sind, enthalten; Pockenvirus-DNA-Impfstoffe, Alphavirus-DNA-Impfstoffe, Filovirus-DNA-Impfstoffe und Zika-Virus-DNA-Impfstoffe.

4. Plasmidbasierte DNA-Impfstoffzusammensetzung nach einem der Ansprüche 1 bis 3, wobei die plasmidbasierte DNA-Impfstoffzusammensetzung bei Kühlung oder bei gefrorener Lagerung stabil ist.

5. Plasmidbasierte DNA-Impfstoffzusammensetzung nach einem der Ansprüche 1 bis 4, wobei die Lipide in Form von Partikeln vorliegen.

6. Plasmidbasierte DNA-Impfstoffzusammensetzung nach Anspruch 5, wobei die Partikel der Lipidformulierung einen mittleren Durchmesser von 80-110 nm und eine Polydispersität von weniger als 0,2 aufweisen.

7. Plasmidbasierte DNA-Impfstoffzusammensetzung nach einem der Ansprüche 1 bis 6, wobei der plasmidbasierte DNA-Impfstoff mit der Lipidformulierung eingekapselt ist.

8. Verfahren zur Formulierung eines plasmidbasierten DNA-Impfstoffs, wobei das Verfahren die Formulierung eines plasmidbasierten DNA-Impfstoffs in einer Lipidformulierung umfasst, die ein Lipid umfasst, ausgewählt aus der Gruppe bestehend aus:

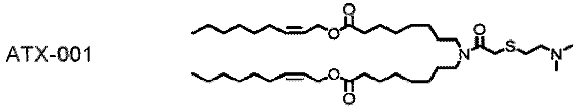

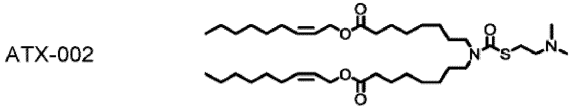

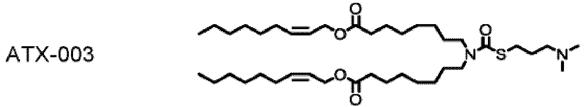

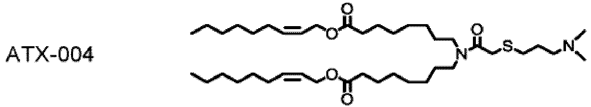

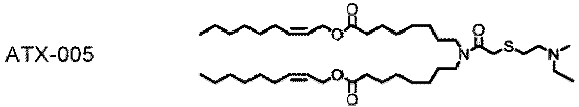

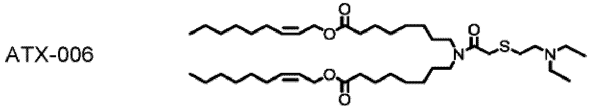

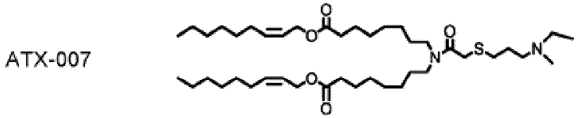

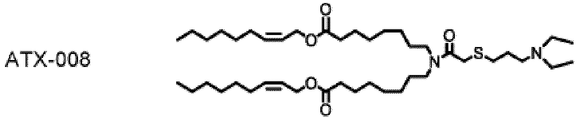

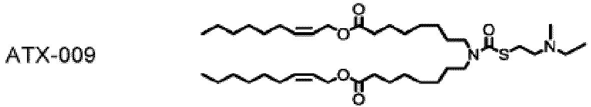

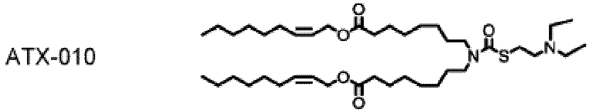

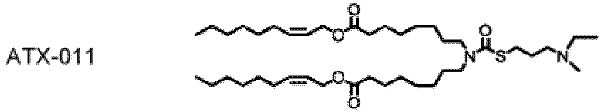

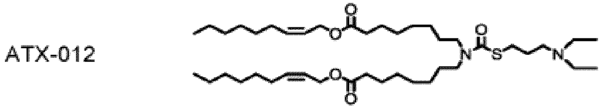

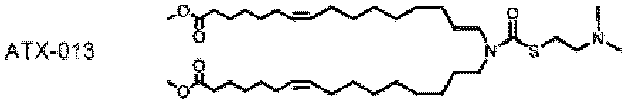

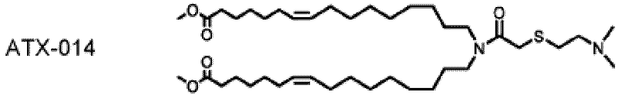

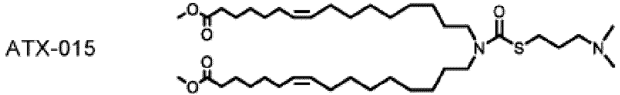

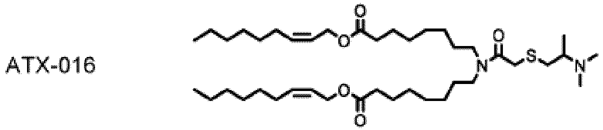

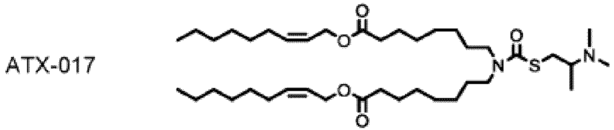

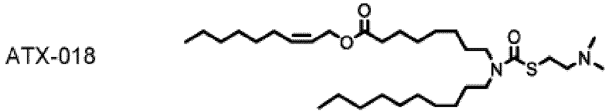

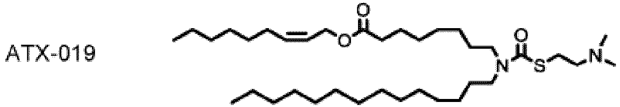

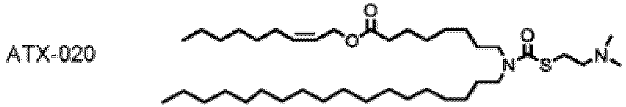

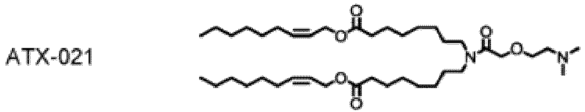

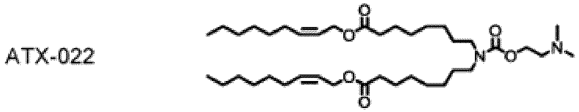

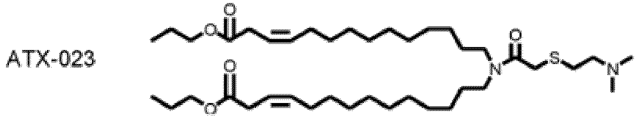

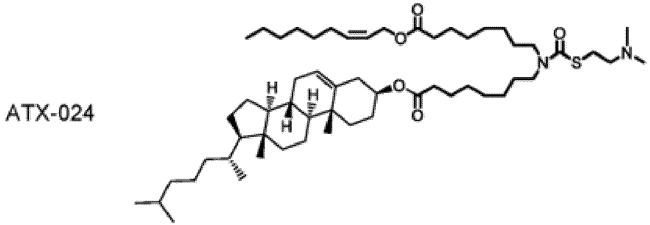

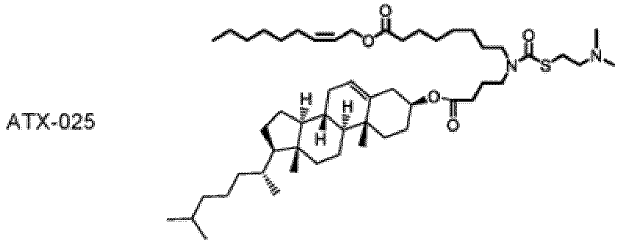

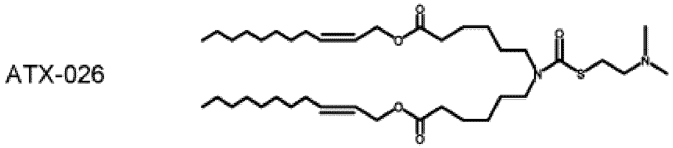

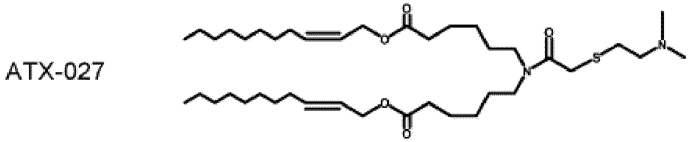

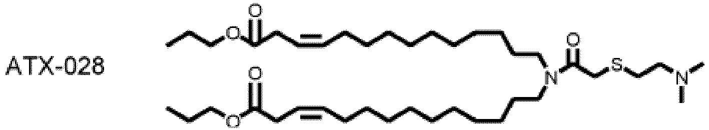

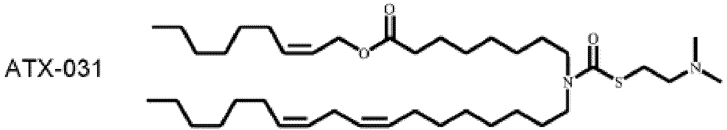

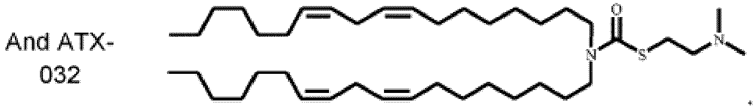

9. Verfahren nach Anspruch 8, wobei die Lipidformulierung ferner einen oder mehrere Lipidhilfsstoffe umfasst, ausgewählt aus der Gruppe bestehend aus 1,2-Distearoyl-sn-glycero-3-phosphocholin, Cholest-5-en-3β-ol und 1,2-Dimyristoyl-rac-glycero-3-methyl-Polyoxyethylen.

10. Verfahren nach Anspruch 8 oder 9, wobei der plasmidbasierte DNA-Impfstoff ausgewählt ist aus der Gruppe bestehend aus Hantavirus-Impfstoffen, die das Andes-Virus, das Sin-Nombre-Virus, das Hantaan-Virus und das Puumala-Virus enthalten; südamerikanische Arenavirus-Impfstoffe, die solche, die gegen das Junin-Virus, das Machup-Virus, das Guanarito-Virus und das Sabia-Virus gerichtet sind, enthalten; Pockenvirus-DNA-Impfstoffe, Alphavirus-DNA-Impfstoffe, Filovirus-DNA-Impfstoffe und Zika-Virus-DNA-Impfstoffe.

11. Verfahren nach einem der Ansprüche 8 bis 10, wobei die Lipide in Form von Partikeln vorliegen.

12. Verfahren nach Anspruch 11, wobei die Partikel der Lipidformulierung einen mittleren Durchmesser von 80-110 nm und eine Polydispersität von weniger als 0,2 aufweisen.

13. Verfahren nach einem der Ansprüche 8 bis 12, wobei der plasmidbasierte DNA-Impfstoff mit der Lipidformulierung eingekapselt ist.

## Revendications

1. Composition de vaccin à ADN plasmidique comprenant une formulation lipidique, ainsi que des immunogènes vaccinaux ou des molécules induisant une réponse immunitaire, la formulation lipidique comprenant au moins un lipide sélectionné dans le groupe constitué par :

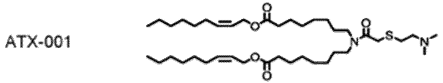

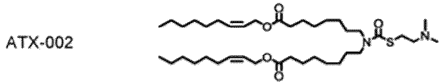

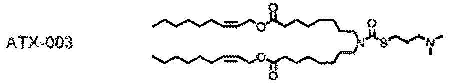

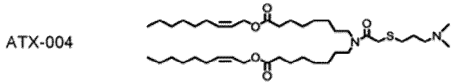

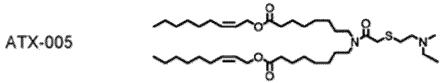

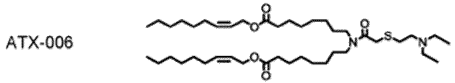

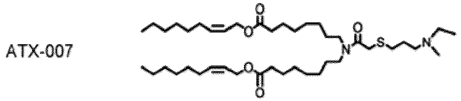

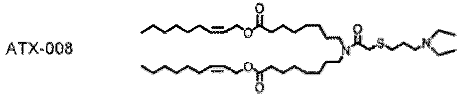

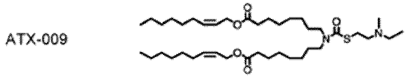

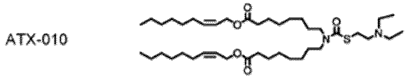

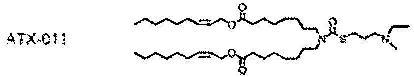

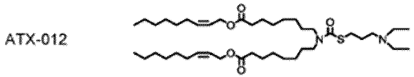

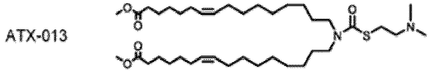

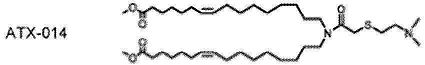

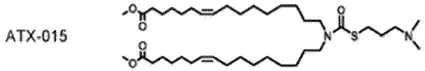

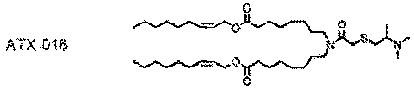

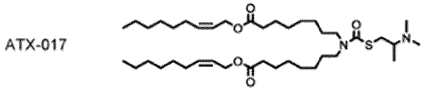

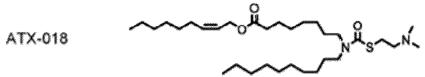

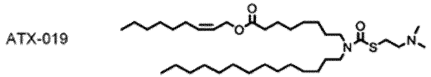

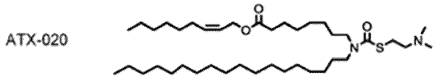

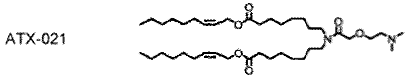

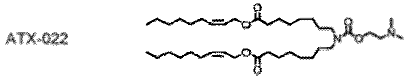

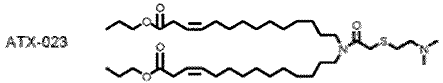

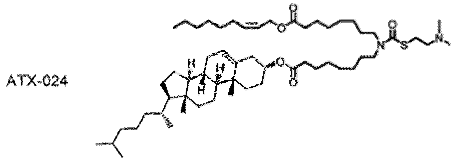

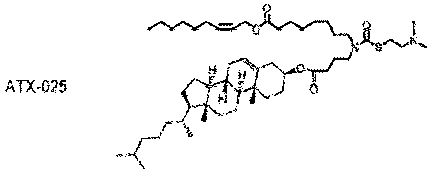

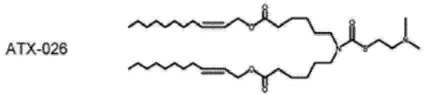

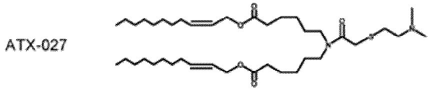

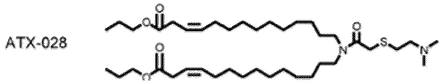

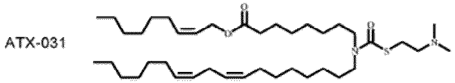

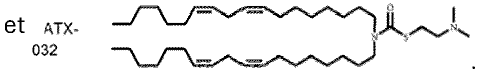

2. Composition de vaccin à ADN plasmidique selon la revendication 1, dans laquelle la formulation lipidique comprend en outre un ou plusieurs excipients lipidiques sélectionnés dans le groupe constitué par 1,2-distéaroyl-sn-glycéro-3-phosphocholine, cholest-5-én-3β-ol et 1,2-dimyristoyl-rac-glycéro-3-méthylpolyoxyéthylène.

3. Composition de vaccin à ADN plasmidique selon la revendication 2, dans laquelle le vaccin à ADN plasmidique est sélectionné dans le groupe constitué par les vaccins contre les hantavirus, dont ceux ciblant le virus des Andes, le virus Sin Nombre, le virus Hantaan et le virus Puumala ; les vaccins contre les arénavirus sud-américains, dont ceux ciblant le virus Junin, le virus Machup, le virus Guanarito et le virus Sabia ; les vaccins à ADN contre les poxvirus, les vaccins à ADN contre les alphavirus, les vaccins à ADN contre les filovirus et les vaccins à ADN contre le virus Zika.

4. Composition de vaccin à ADN plasmidique selon l'une quelconque des revendications 1 à 3, ladite composition de vaccin à ADN plasmidique étant stable lorsqu'elle est réfrigérée ou conservée à l'état congelé.

5. Composition de vaccin à ADN plasmidique selon l'une quelconque des revendications 1 à 4, dans laquelle les lipides se présentent sous forme de particules.

6. Composition de vaccin à ADN plasmidique selon la revendication 5, dans laquelle les particules de la formulation lipidique présentent un diamètre moyen de 80 à 110 nm et une polydispersité inférieure à 0,2.

7. Composition de vaccin à ADN plasmidique selon l'une quelconque des revendications 1 à 6, dans laquelle le vaccin à ADN plasmidique est encapsulé dans la formulation lipidique.

8. Procédé de formulation d'un vaccin à ADN plasmidique, le procédé comprenant la formulation d'un vaccin à ADN plasmidique dans une formulation lipidique comprenant un lipide sélectionné dans le groupe constitué par :

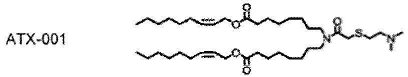

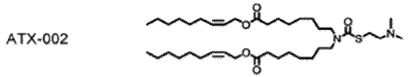

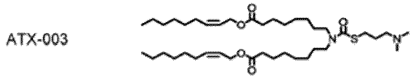

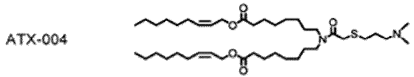

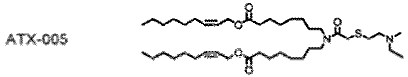

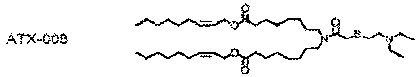

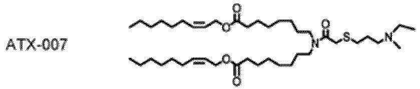

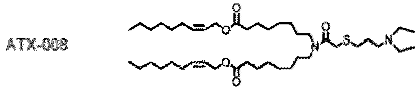

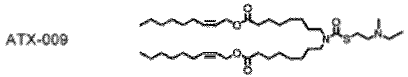

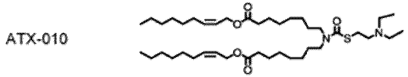

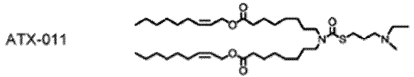

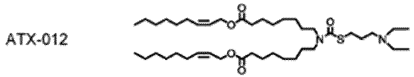

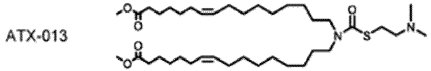

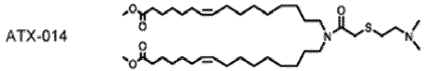

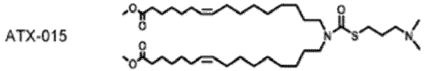

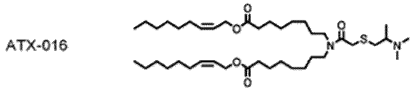

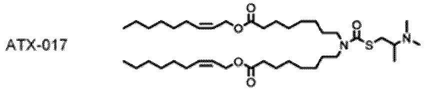

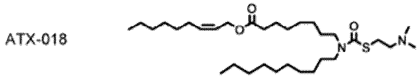

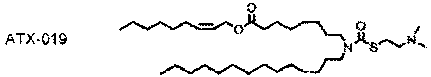

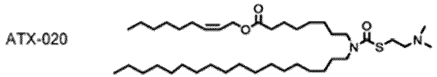

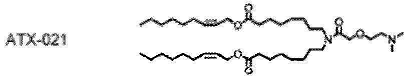

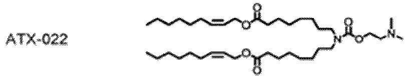

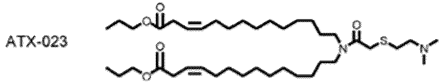

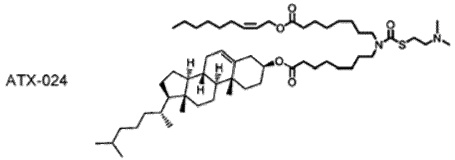

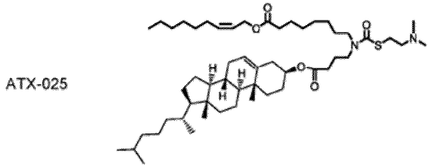

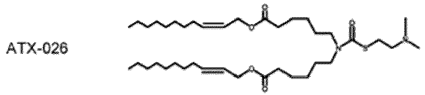

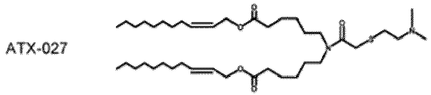

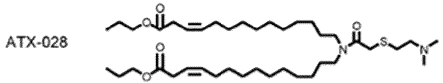

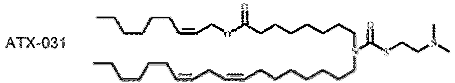

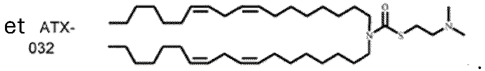

9. Procédé selon la revendication 8, dans lequel la formulation lipidique comprend en outre un ou plusieurs excipients lipidiques sélectionnés dans le groupe constitué par 1,2-distéaroyl-sn-glycéro-3-phosphocholine, cholest-5-én-3β-ol et 1,2-dimyristoyl-rac-glycéro-3-méthylpolyoxyéthylène.

10. Procédé selon la revendication 8 ou 9, dans lequel le vaccin à ADN plasmidique est sélectionné dans le groupe constitué par les vaccins contre les hantavirus, dont ceux ciblant le virus des Andes, le virus Sin Nombre, le virus Hantaan et le virus Puumala ; les vaccins contre les arénavirus sud-américains, dont ceux ciblant le virus Junin, le virus Machup, le virus Guanarito et le virus Sabia ; les vaccins à ADN contre les poxvirus, les vaccins à ADN contre les alphavirus, les vaccins à ADN contre les filovirus et les vaccins à ADN contre le virus Zika.

11. Procédé selon l'une quelconque des revendications 8 à 10, dans lequel les lipides se présentent sous forme de particules.

12. Procédé selon la revendication 11, dans lequel les particules de la formulation lipidique présentent un diamètre moyen de 80 à 110 nm et une polydispersité inférieure à 0,2.

13. Procédé selon l'une quelconque des revendications 8 à 12, dans lequel le vaccin à ADN plasmidique est encapsulé dans la formulation lipidique.
